# EUROPÄISCHE PATENTSCHRIFT

(11) **EP 1 937 221 B1**
(45) Veröffentlichungstag und Bekanntmachung des Hinweises auf die Patenterteilung: **11.02.2009**
(21) Anmeldenummer: 06805947.6
(22) Anmeldetag: 29.09.2006
(51) Int. Cl.: A61K 9/20, A61K 31/551

(54) **RETARDFORMULIERUNG FÜR PRALNACASAN**
RETARD FORMULATION FOR PRALNACASAN
FORMULATION A EFFET RETARD POUR PRALNACASAN

(30) Priorität: 08.10.2005 DE 102005048293
(43) Veröffentlichungstag der Anmeldung: 02.07.2008
(73) Patentinhaber: Sanofi-Aventis Deutschland GmbH, 65929 Frankfurt am Main (DE)
(72) Erfinder: SOENNICHSEN, Caren, 65926 Frankfurt am Main (DE); WESCH, Roland, 65926 Frankfurt am Main (DE); MEIER, Heiko, 65926 Frankfurt am Main (DE)
(86) Internationale Anmeldenummer: PCT/EP2006/009466
(87) Internationale Veröffentlichungsnummer: WO 2007/042160

(56) Entgegenhaltungen:
- WO-A-95/01781
- GB-A- 2 230 185
- US-A1- 2005 013 863
- US-A1- 2005 053 653

## Beschreibung

Die Erfindung betrifft eine Retardformulierung enthaltend Pralnacasan.

Pralnacasan (1 S, 9S (RS, 3S) N-(2-Ethoxy-5-oxo-tetrafuran-3-yl)-6,10-dioxo-9-(isochinolin-1-oyl-amino)-1, 2, 3, 4, 7, 8, 9, 10-octahydro-6-H-pyridazino [1, 2-a] [1, 2] diazepin-1-carboxamide sowie seine Salze und Derivate sind aus der WO 97/22619 zur Behandlung z. B. von Autoimmunkrankheiten, Rheumatoider Arthritis, Osteoarthritis, Diabetes Typ I und Typ 11 und Psoriasis bekannt. Die dabei wirksame Verbindung ist die offenkettige Verbindung 3S(1S, 9S) 3-[6, 10-dioxo-9 (isochinolin-1-oylamino)-1, 2, ,3; 4, 7, 8, 9, 10-octahydro-6H-pyridazino [1, 2-a] [1, 2] diazepin-1-carboxamido]-4-oxobutan Säure, während Pralnacasan das Prodrug bzw. Arzneimittel hierzu ist. In der WO 97/22619 wird auch eine Standardtabelle zur schnellen Freisetzung des Prodrugs beschrieben.

Aufgabe der Erfindung war es, eine Retardtablette bereit zu stellen, die es ermöglicht, bei nur einer zweimal täglichen Gabe eine gleichmäßige Abgabe von Pralnacasan und/oder seinen Salzen und/oder Derivaten bei gleichzeitig verringerter Gesamttagesdosis, verglichen mit der Standardtablette, zu ermöglichen.

Gelöst wird die Aufgabe durch eine Retardtablette bestehend aus mindestens zwei Schichten, wobei mindestens eine Schicht das Pralnacasan und/oder seine Salze und/oder seine Derivate schnell abgibt (Initialdosis) und mindestens eine zweite, Schicht, die Pralnacasan und/oder seine Salze und/oder seine Derivate retardiert abgibt (Erhaltungsdosis). Die Schichten können sandwichartig übereinander liegen, aber auch als Kern und Mantel angeordnet sein.

Unter, "schneller' Abgabe wird verstanden, dass mindestens 60 % von Pralnacasan bzw. dessen Salze und/oder Derivate bereits innerhalb von 30 Minuten aus der Tablette freigesetzt sind. Unter "retardierender" Abgabe wird verstanden, dass mindestens 90 % von Pralnacasan bzw. dessen Salze und/oder Derivate nach 180 Minuten freigesetzt sind. Dabei wird das Absorptionsfenster von 6 h auf 8 h verlängert. In konventionellen, das Arzneimittel schnell abgebenden Tabletten, ist Pralnacasan nur ungenügend wirksam oder müsste so hoch dosiert werden, dass unerwünschte Nebenwirkungen zu befürchten sind. Eine dreimal tägliche Gabe, welche eine einmalig hohe Dosierung vermeiden würde, ist aber bezüglich der Patientencompliance nicht erstrebenswert. Andererseits ist eine übliche Retardtablette für die zweimal tägliche Gabe (Arzneimittelfreigabe über 2x6 = 12h) ebenfalls unbefriedigend, weil erstens die Arzneimittelfreisetzung zu langsam verläuft, so dass anfänglich kein ausreichend hoher, therapeutisch wirksamer Blutspiegel erreicht wird, und zweitens der erst im unterem Darmabschnitt (Dickdarm) freigesetzte Arzneimittelanteil dort nicht mehr resorbiert wird, also unwirksam bleibt. Eine Kombination von konventioneller Tablette mit Retardtablette schien aber auch nicht erstrebenswert, weil sich normalerweise beide Teile einer solchen Tablette negativ beeinflussen.

Bei der erfindungsmäßigen, mindestens zweischichtigen Retardtablette zeigt sich überraschend, dass die schnelle Abgabe des Arzneimittels nicht durch den Retardteil negativ beeinflusst wird, d. h. die Schicht(en) mit dem Initialanteil, ermöglicht(en) einen Wirkungseintritt der erfindungsmäßigen Retardtablette, der überraschenderweise nicht langsamer als der der Standardtablette ist, und der Retardanteil wird hinreichend lange und vollständig resorbiert, obwohl dieser Anteil ausschließlich im oberen GT-Trakt resorbiert wird, d. h. der Retardanteil wird nicht nur nicht durch den Initialanteil negativ beeinflusst, sondern überraschenderweise sogar positiv beeinflusst. Insbesondere diese Wirkungsweise war für eine Kombinationstablette nicht vorauszusehen.

Mit der erfindungsgemäßen Retardtablette werden überraschender Weise große Blutspiegelspitzen verhindert, die üblicherweise für toxische Nebenwirkungen eines Arzneimittels verantwortlich sind. Durch den Einsatz der erfindungsgemäßen Retardtablette wird die Patientencompliance verbessert, weil weniger Arzneimittel pro Tag im größeren Dosen eingenommen werden muss. Trotz mehrerer Schichten des Arzneimittels, ist die erforderliche Retardtablette nicht voluminöser als eine Standardtablette. Das ist vor allem für ältere Patienten wichtig und vorteilhaft.

Die erfindungsgemäße Retardtablette besteht bevorzugt aus zwei Schichten, die sowohl als Kern und Mantel, bevorzugt konzentrisch als Kugel oder als Elipsoid, als auch als übereinander liegende Schichten angeordnet sind. Bei der Schichttablette kann es sich um eine Tablette mit kreisrundem oder ovalem Grundriss handeln. In der Kern-Mantel-Anordnung befindet sich die Initialdosis bevorzugt im Außenmantel, die Retarddosis im Kernbereich.

Die bevorzugt zwei Schichten der erfindungsgemäßen Tablette können jeweils neben dem Arzneimittel jeweils einen oder mehrere Füllstoffe, bevorzugt zwei oder drei, und ein oder mehrere Bindemittel bevorzugt ist ein Bindemittel, und ein oder mehrere, bevorzugt ein, Schmiermittel enthalten. Die Füllstoffe, Bindemittel und Schmiermittel können in den jeweiligen Schichten pro Tablette unterschiedlich sein. Auch deren Anzahl kann jeweils unterschieden werden. Bevorzugt sind in der Retardschicht drei unterschiedliche Füllstoffe enthalten, in der schnellfreigebenden Schicht dagegen zwei unterschiedliche Füllstoffe.

Von den Bindemitteln und Schmiermitteln sind in den Schichten bevorzugt die gleichen enthalten.

Die Retardschicht enthält darüber hinaus zusätzlich einen oder mehrere Gelbilder, insbesondere, wenn die Retardschicht die äußere Schicht der Tablette darstellt. Bevorzugt ist ein Gelbilder.

Die schnell freisetzende Schicht enthält zusätzlich ein oder mehrere Zerfallshilfsmittel, bevorzugt ist ein Zerfallshilfsmittel.

Beispiele für Füllstoffe sind Maisstärke, Phosphate, wie Calciumphosphat, Lactose, z.B. Lactose D80, Saccharose, wie Mannitol oder mikrokristalline Cellulosen z.B. Typ 102. Bevorzugt sind Lactose, Mannitol oder mikrokristalline Cellulose . Die Füllstoffe sind in den jeweiligen Schichten im Bereich 0-400 mg/Schicht, bevorzugt 10 - 200, besonders bevorzugt 20-100 erhalten. Sollten pro Schicht zwei oder drei Füllstoffe enthalten sein, so sind diese im Verhältnis von ca. 2:1 bzw. 2:1:1 enthalten. Sollten zwei Füllstoffe enthalten sein, so bevorzugt Lactose D80 und Mannitol oder mikrokristalline Cellulose.
Sollten drei Füllstoffe enthalten sein, so bevorzugt Laktose D80, mikrokristalline Cellulose und Mannitol.
Bevorzugt enthält die Retardschicht drei Füllstoffe, die schnellfreisetzende Schicht zwei Füllstoffe.

Von den Bindemitteln kommen allgemein Celluloseether oder Polyvinylpyrrolidon in Frage. Bevorzugt ist Hydroxypropylcellulose im Bereich 5 - 30 mg/Schicht, bevorzugt 10-20 mg/Schicht.

Schmiermittel können die dem Fachmann bekannten Stearate, wie Magnesiumstearat und Fumarate, z.B. Natriumstearylfumarat sein. Pro Schicht sind davon 5-20 mg vorhanden, in der schnellfreisetzenden Schicht sind bevorzugt 1-5 mg Schmiermittel vorhanden.

Zusätzlich sind in der Retardschicht ein oder mehrere Gelbilder, wie z. B.
Hydroxypropylmethylcellulose oder Carrageen, bevorzugt
Hydroxypropylmethylcellulose im Bereich von 20-100 mg/Schicht, besonders bevorzugt 30-50 mg/Schicht enthalten. In der schnellfreisetzenden Schicht ist zusätzlich mindestens ein Zufallshilfsmittel, wie z. B. Croscarmellose oder Crospovidon, bevorzugt Croscarmellose, im Bereich von 5-20 mg/Schicht, bevorzugt 6-15 mg/Schicht enthalten.

Grundsätzlich sind alle Bestandteile der Tablette, die in beiden Schichten vorkommen, im Verhältnis 1,5-1,0 bis 3,0-1,0, bevorzugt 2,0-1,0 in der Retardschicht zur schnellfreisetzenden Schicht enthalten.

Dies gilt auch für als Arzneimittel: Die Retardschicht enthält 200-600 mg Arzneimittel/Schicht, bevorzugt 300-500 mg/Schicht. Die schnellfreisetzende Schicht enthält 50-400 mg Arzneimittel/Schicht, bevorzugt 100-300 mg/Schicht.

Bevorzugt ist das Verhältnis 400:200 mg/Schicht. Als Arzneimittel kommt Pralnacasan und/oder dessen Derivate und die jeweiligen Salze als auch die jeweils wirksamen Verbindungen (freie Säuren) in Frage .

Auch können Mischungen aus Pralnacasan und/oder dessen Salze und/oder dessen Derivate bzw. Mischungen der jeweils freien Säuren vorliegen. Auch Mischungen aus Pralnacasan oder dessen Derivate mit den freien Säuren sind einsetzbar.

Die Herstellung der erfindungemäßen Retardtablette erfolgt dadurch, dass das Arzneimittel granuliert wird. Dieses Granulat wird mit den zusätzlichen Hilfsstoffen wie Füllstoff, Bindmittel und Schmiermittel vermischt. Speziell für die Retardschicht wird schließlich der Gelbilder zugegeben, bei der schnellfreisetzenden Schicht wird das Zerfallshilfsmittel dazugegeben. Die beiden Schichten werden miteinander zu einer Tablette verpresst.

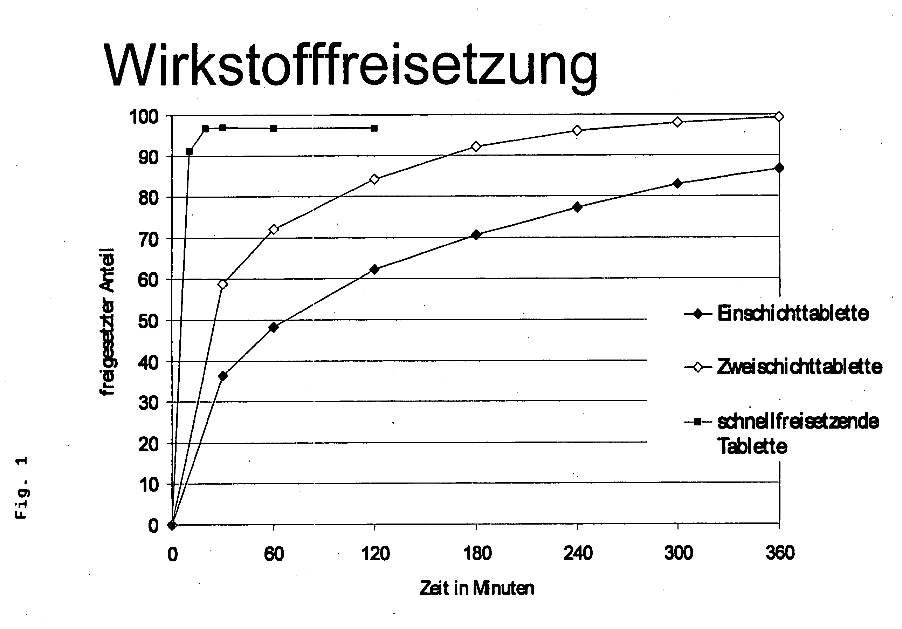

In Figur 1 ist die Wirkstofffreisetzung der erfindungsgemäßen Retardformulierung (Zweischichttablette) mit einer schnellfreisetzenden Tablette und einer Retardtablette (Einschichttablette) verglichen.

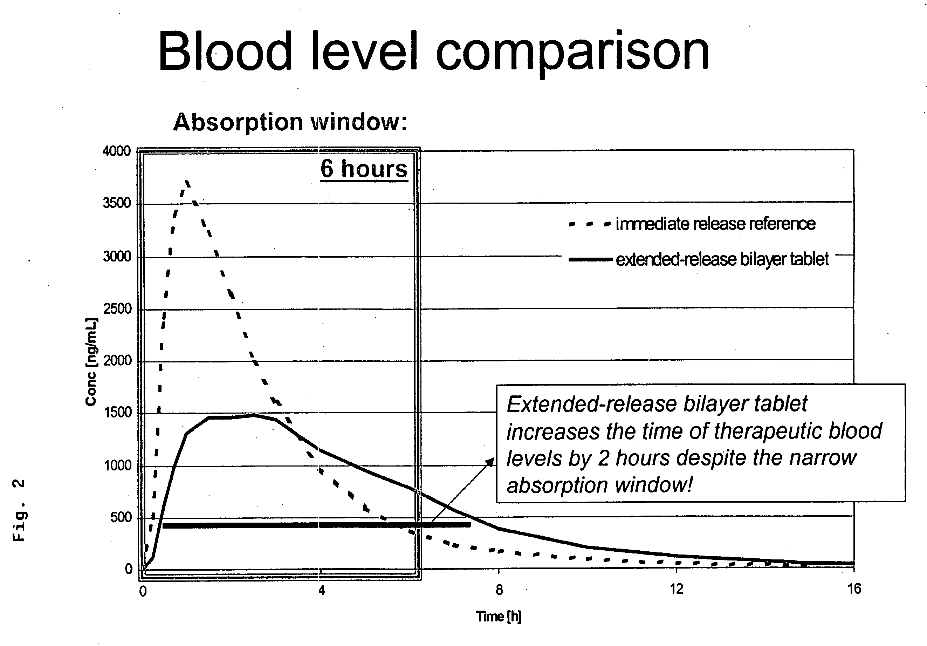

Überraschenderweise zeigt sich, dass bis der erfindungsmäßigen Tablette mindestens 60 % des Arzneimittels bereits nach 30 Minuten freigesetzt ist und mindestens 90 % nach 180 Minuten. Gemäß Figur 2 verlängert die erfindungsgemäße Tablette damit die therapeutische Konzentration im Blut um 2 Stunden über das enge Absorptionsfenster von 6 Stunden hinaus, verglichen mit der schnellfreisetzenden Tablette.

Die erfindungsgemäße Retardformulierung wird bevorzugt zur Behandlung von Autoimmunkrankheiten, Diabetes Typ I und Typ 11, Rheumatoider Arthritis, Osteoarthritis und Psoriasis. Generell können alle in der WO 97/22619 genannten Erkrankungen mit der erfindungsmäßigen Retardtabletten behandelt werden.

Die erfindungsgemäße Retardformulierung kann oral in Form von Kapseln und Tabletten verabreicht werden. Bevorzugt wie die Retardformulierung einmal pro Tag verabreicht. Die Dosis des Arzneimittels liegt bei 0,01 bis 100 mg/kg Körpergewicht pro Tag, bevorzugt bei 1 bis 50 mg/kg Körpergewicht pro Tag. Die Erfindung soll deutlich gemacht werden an folgenden Beispielen (Zweischichttablette):

| | Retardschicht |
|---|---|
| Pralnacasan | 400 |
| Lactose D 80 | 62 |
| Hydroxypropylcellulose | 18 |
| Mikrokristalline Cellulose Typ 102 | 30 |
| Hydroxypropylmethylcellulose 4000mPas | 40 |
| Mannitol | 30 |
| Magnesiumstearat | 10 |
| SUMME | 590 |

| | schnellfreisetzende Schicht |
|---|---|
| Pralnacasan | 200 |
| Lactose D 80 | 31 |
| Croscarmellose | 10 |
| Hydroxypropylcellulose | 9 |
| Mikrokristalline Cellulose Typ 102 | 15 |
| Magnesiumstearat | 1,5 |
| SUMME | 266,5 |

| | Gesamte Tablette |
|---|---|
| HMR 3480 | 600 |
| Lactose D 80 | 93 |
| Hydroxypropylcellulose | 27 |
| Croscarmellose | 10 |
| Mikrokristalline Cellulose Typ 102 | 45 |
| Hydroxypropylmethylcellulose 4000mPas | 40 |
| Mannitol | 30 |
| Magnesiumstearat | 11,5 |
| SUMME | 856,5 |

| | |
|---|---|
| Angaben in mg/Tablette | |

## Patentansprüche

1. Retardtablette bestehend aus mindestens zwei Schichten, **dadurch gekennzeichnet, dass** mindestens eine Schicht das Arzneimittels 1 S, 9S (RS, 3S) N-(2-Ethoxy-5-oxo-tetrafuran-3-yl)-6,10-dioxo-9-(isochinolin-1-oyl-amino)-1, 2, 3, 4, 7, 8, 9, 10-octahydro-6-H-pyridazino [1, 2-a] [1, 2] diazepin-1-carboxamide und/oder dessen Salze oder Derivate und/oder die freien Säuren davon, schnell abgibt und mindestens eine Schicht das Arzneimittels 1 S, 9S (RS, 3S) N-(2-Ethoxy-5-oxo-tetrafuran-3-yl)-6,10-dioxo-9-(isochinolin-1-oyl-amino)-1, 2, 3, 4, 7, 8, 9, 10-octahydro-6-H-pyridazino [1, 2-a] [1, 2] diazepin-1-carboxamide und/oder dessen Salze oder Derivate und/oder die freien Säuren davon retardiert abgibt.

2. Retardtablette nach Anspruch 1, **dadurch gekennzeichnet, dass** die Tablette aus zwei Schichten besteht, und diese schichtförmig oder als Kern und Mantel angeordnet sind.

3. Retardtablette nach einem oder mehreren der Ansprüche 1 und 2, **dadurch gekennzeichnet, dass** die Retardschicht als Kern einer Kern-Mantel-Anordnung vorliegt.

4. Retardtablette nach einem oder mehreren der Ansprüche 1 bis 3, **dadurch gekennzeichnet, dass** beide Schichten mindestens einen Füllstoff, mindestens ein Bindemittel und mindestens ein Schmiermittel enthalten.

5. Retardtablette nach einem oder mehreren der Ansprüche 1 bis 4, **dadurch gekennzeichnet, dass** die schnellabgebende Schicht zusätzlich mindestens ein Zerfallshilfsmittel enthält.

6. Retardtablette nach einem oder mehreren der Ansprüche 1 bis 4, **dadurch gekennzeichnet, dass** die retardierende Schicht mindestens einem Gelbilder enthält.

7. Retardtablette nach einem oder mehreren der Ansprüche 1 bis 6, **dadurch gekennzeichnet, dass** mindestens 60 % des Arzneimittels nach höchstens 30 Minuten und mindestens 90 % nach 180 Minuten aus der Tablette freigesetzt ist.

8. Retardtablette nach einem oder mehrere der Ansprüche 1 bis 7 zur Behandlung von Autoimmunkrankheiten, Diabetes Typ I und Typ II, rheumatoider Arthritis, Osteoarthritis, und/oder Psoriasis.

## Claims

1. An extended release tablet comprising of at least two layers, wherein at least one layer delivers the pharmaceutical agent 1S, 9S (RS, 3S) N-(2-ethoxy-5-oxo-tetrafuran-3-yl)-6,10-dioxo-9-(isoquinolin-1-oylamino)-1, 2, 3, 4, 7, 8, 9, 10-octahydro-6-H-pyridazino [1, 2-a] [1, 2] diazepine-1-carboxamide and/or its salts or derivatives and/or the free acids thereof rapidly, and at least one layer delivers the pharmaceutical agent 1S, 9s (RS, 3S) N-(2-ethoxy-5-oxo-tetrafuran-3-yl)-6,10-dioxo-9-(isoquinolin-1-oylamino)-1, 2, 3, 4, 7, 8, 9, 10-octahydro-6-H-pyridazino [1, 2-a] [1, 2] diazepine-1-carboxamide and/or its salts or derivatives and/or the free acids thereof in extended fashion.

2. The extended release tablet as claimed in claim 1, wherein the tablet consists of two layers, and these are disposed in layer form or as core and shell.

3. The extended release tablet as claimed in one or more of claims 1 and 2, wherein the extended release layer is present as the core of a core-shell arrangement.

4. The extended release tablet as claimed in one or more of claims 1 to 3, wherein both layers comprise at least one filler, at least one binder and at least one lubricant.

5. The extended release tablet as claimed in one or more of claims 1 to 4, wherein the rapid delivery layer additionally comprises at least one disintegrant.

6. The extended release tablet as claimed in one or more of claims 1 to 4, wherein the extended release layer comprises at least one gel former.

7. The extended release tablet as claimed in one or more of claims 1 to 6, wherein at least 60% of the pharmaceutical agent is released after not more than 30 minutes, and at least 90% after 180 minutes, from the tablet.

8. The extended release tablet as claimed in one or more of claims 1 to 7 for the treatment of autoimmune diseases, type I and type II diabetes, rheumatoid arthritis, osteoarthritis, and/or psoriasis.

## Revendications

1. Comprimé à effet de retard contenu par au moins deux couches, **caractérisé en ce qu'**au moins une couche libère rapidement le médicament 1S,9S(RS,3S)N-(2-éthoxy-5-oxotétrafuran-3-yl)-6,10-dioxo-9-(isoquinoléin-1-oylamino)-1,2,3,4,7,8,9,10-octahydro-6-H-pyridazino[1,2-a][1,2]diazépine-1-carboxamide et/ou ses sels ou dérivés et/ou les acides libres de ceux-ci et au moins une couche libère de manière retardée le médicament 1S,9S(RS,3S)N-(2-éthoxy-5-oxotétrafuran-3-yl)-6,10-dioxo-9-(isoquinoléin-1-oylamino)-1,2,3,4,7,8,9,10-octahydro-6-H-pyridazino[1,2-a][1,2]diazépine-1-carboxamide et/ou ses sels ou dérivés et/ou les acides libres de ceux-ci.

2. Comprimé à effet de retard selon la revendication 1, **caractérisé en ce que** le comprimé est constitué par deux couches et celles-ci sont disposées en forme de couche ou sous forme de noyau et d'enveloppe.

3. Comprimé à effet de retard selon l'une ou plusieurs des revendications 1 et 2, **caractérisé en ce que** la couche de retard se trouve sous forme de noyau d'une disposition à noyau-enveloppe.

4. Comprimé à effet de retard selon l'une ou plusieurs des revendications 1 à 3, **caractérisé en ce que** les deux couches contiennent au moins une charge, au moins un liant et au moins un lubrifiant.

5. Comprimé à effet de retard selon l'une ou plusieurs des revendications 1 à 4, **caractérisé en ce que** la couche à libération rapide contient en outre au moins un adjuvant de décomposition.

6. Comprimé à effet de retard selon l'une ou plusieurs des revendications 1 à 4, **caractérisé en ce que** la couche à effet de retard contient au moins un agent gélifiant.

7. Comprimé à effet de retard selon l'une ou plusieurs des revendications 1 à 6, **caractérisé en ce qu'**au moins 60% du médicament sont libérés du comprimé après au plus 30 minutes et au moins 90% sont libérés après 180 minutes.

8. Comprimé à effet de retard selon l'une ou plusieurs des revendications 1 à 7 pour le traitement de maladies auto-immunitaires, du diabète de type I et de type II, de l'arthrite rhumatoïde, de l'ostéoarthrite, et/ou du psoriasis.
